# EUROPÄISCHE PATENTSCHRIFT

(11) **EP 1 993 479 B1**
(45) Veröffentlichungstag und Bekanntmachung des Hinweises auf die Patenterteilung: **29.09.2010**
(21) Anmeldenummer: 07710520.3
(22) Anmeldetag: 16.03.2007
(51) Int. Cl.: A61F 2/14

(54) **HORNHAUTIMPLANTAT**
CORNEA IMPLANT
IMPLANT CORNÉEN

(30) Priorität: 16.03.2006 AT 4282006
(43) Veröffentlichungstag der Anmeldung: 26.11.2008
(73) Patentinhaber: Daxer, Albert, A-4020 Linz (AT)
(72) Erfinder: Daxer, Albert, A-4020 Linz (AT)
(74) Vertreter: KLIMENT & HENHAPEL
(86) Internationale Anmeldenummer: PCT/AT2007/000130
(87) Internationale Veröffentlichungsnummer: WO 2007/104068

(56) Entgegenhaltungen:
- WO-A-01/49334
- WO-A-93/14703
- US-A1- 2001 004 708
- US-A1- 2003 033 015
- US-A1- 2005 119 738

## Beschreibung

### GEBIET DER ERFINDUNG

Die vorliegende Erfindung bezieht sich auf ein ringförmiges Hornhautimplantat zur Einbringung in eine Hornhauttasche des menschlichen Auges über einen schmalen, vorzugsweise tunnelförmigen Zugang, wobei die Endgestalt des Hornhautimplantats von der zu korrigierenden Fehlsichtigkeit abhängt.

### STAND DER TECHNIK

Der optische Apparat des Auges zur Abbildung der Umwelt besteht im Wesentlichen aus der Hornhaut und der hinter der Iris gelegenen Linse. Dieser optische Apparat des Auges besitzt eine Gesamtbrechkraft von ungefähr 60 Dioptrien, wobei die Grenzfläche zwischen Hornhaut und Luft, also die äußere Begrenzung des Auges, den Hauptbeitrag mit etwa 40 Dioptrien liefert. Diese Brechkraft der Hornhaut ist im Wesentlichen indirekt proportional zum Radius der Hornhautvorderfläche (Grenzfläche zwischen Hornhaut und Luft). Durch Veränderung des Hornhautradius lässt sich somit auch die Brechkraft des Auges verändern. So führt etwa eine Vergrößerung des (zentralen) Hornhautradius zu einer Abnahme der Brechkraft, was man sich chirurgisch zur operativen Korrektur bei Kurzsichtigkeiten zu Nutze macht. Dabei wird etwa bei den Lasermethoden (LASIK, LASEK, etc.) im Bereich der zentralen Hornhaut mehr Gewebe entfernt als in der Peripherie.

Bei der LASIK Methode beispielsweise wird ein "flap" in die Hornhaut geschnitten. Das ist eine lamelläre Abkappung der

Hornhaut in einer bestimmten Tiefe. Der erhebliche Nachteil derartiger "flaps", ist, dass dadurch die biomechanische Stabilität der Hornhaut erheblich beeinträchtigt wird. Im Besonderen wächst dieser "flap" nicht mehr vollständig am darunterliegenden Hornhautgewebe an. Dadurch verringert sich die biomechanisch wirksame Querschnittsfläche der Hornhaut um genau jenen Betrag, der der Dicke des "flaps" entspricht. Es ist daher wünschenswert, eine Korrektur im Inneren der Hornhaut vorzunehmen, ohne eine derart massive Störung der Biomechanik, wie sie durch einen "flap" hervorrufen wird, in Kauf nehmen zu müssen. Außerdem sind die erwähnten Lasermethoden nur bis etwa 10 Dioptrien Kurzsichtigkeit geeignet.

Um diese Nachteile zu verhindern, sind Verfahren entwickelt worden, bei welchen Hornhautimplantate zum Einsatz kommen, die eine Verformung der Hornhaut in dem Sinne bewirken, dass der Hornhautradius durch Volumsaddition vergrößert wird, was zu einer Abnahme der Brechkraft und damit zur Korrektur von Kurzsichtigkeit führt.

Hornhautimplantate sind meist ringförmig ausgebildet, wobei ganz Ringe (offen oder geschlossen) oder geteilte Ringe (z.B. Ringsegmente) zum Einsatz kommen.

Aus der US 2005/0119738 A1 ist beispielsweise der Einsatz eines solchen Hornhautimplantats unter einem "flap" bekannt. Sie beschreibt ein Ringimplantat zur Einbringung in die Hornhaut unter einen Flap. Dabei ist ein zentraler Teil des Hornhautimplantates direkt optisch wirksam, d.h. er muss, um seine Aufgabe zu erfüllen, Teil der optischen Zone sein und von den abzubildenden Lichtstrahlen durchdrungen werden. Die Aufgabe des Hornhautimplantates ist die Ausbildung einer multifokalen Hornhautoberfläche, wobei der Bereich des zentralen Loches im Hornhautimplantat für das Sehen in der Nähe und jener Bereich, der zusätzlich den inneren Teil des Hornhautimplantates mit einschließt (optische Zone) für das Sehen in der Ferne zuständig ist. Um diese gestellte Aufgabe überhaupt lösen zu können, muss die optische Zone (also einschließlich des inneren Teils des Hornhautimplantats) innerhalb des Pupillendurchmessers des Auges sein, der meist zwischen 2 und 5 mm liegt. Dies erfordert, dass das innere Loch im Hornhautimplantat nicht wesentlich größer als 2 mm sein darf. Des weiteren muss aber auch der innere Teil des Hornhautimplantates, der direkt an der optischen Abbildung beim Blick in die Ferne beitragen soll, eine Mindestbreite aufweisen, die unter Berücksichtigung der optischen Gesetze deutlich über 1 mm (2 mm) betragen muss um wirksam zu sein. Dazu kommt noch der äußere Teil des Hornhautimplantates. Dadurch besteht das erhebliche Problem, dass es durch die doch sehr wesentliche Materialbreite von weit über 1 mm zu Problemen mit der Sauerstoff- und Nährstoffversorgung führen kann. Unter Verwendung des angegebenen mikroporösen Hydrogels kann zwar dieses Problem der Nährstoffversorgung gemildert werden, aber dann entsteht durch die Weichheit des Ringes das Problem der Stabilität und Stabilisierung der Ringgeometrie.

Außerdem besitzt das Hornhautimplantat sowohl am inneren Rand, als auch am äußeren Rand keine spitzes Ende, sondern eine endliche Randdicke von vorzugsweise 10 µm. Diese Randkanten sind insofern von erheblichem Nachteil, da es dort zu Ablagerungen kommen kann, was zu Sehbeeinträchtigungen führen kann.

Die Erfahrungen mit multifokalen Abbildungen, sowohl bei der Kontaktlinsenanpassung, als auch bei der Implantation von Intraokularlinsen bei Staroperationen zeigen, dass dies nur von einem geringen Teil der Patienten toleriert wird. Die Mehrzahl empfindet die dadurch bewirkte simultane "Verschmierung" des Brennpunktes entlang der optischen Achse eher als unangenehm denn als vorteilhaft. Außerdem ist bei der vorliegenden Konstruktion zu erwarten, dass die Patienten den zentralen Teil des Hornhautimplantates und dabei, trotz an die Umgebung angeglichenen Brechungsindex, insbesondere den inneren Rand, der ja innerhalb der Pupillenweite sein muss, als störend empfinden.

Die Einbringung eines Hornhautimplantates in die Hornhaut unter einen "flap" hat somit erhebliche Nachteile, insbesondere eine signifikante Beeinträchtigung der Stabilität der Hornhaut durch den lamellären Schnitt in die Hornhaut zur Erzeugung des "flap". Diese Beeinträchtigung der Stabilität der Hornhaut ist aus den Erfahrungen mit LASIK Operationen bekannt, wo ein gleichartiger "flap" erzeugt werden muss.

Ringförmige Hornhautimplantate kommen aber auch bei Verfahren zum Einsatz, welche eine zirkuläre Inzission in der Hornhautoberfläche vorsehen. So beschreibt beispielsweise die GB 2095119 A eine solche zirkuläre Inzission von der Hornhautoberfläche in das Hornhautgewebe, in welche ein Ring mit ca. 8 mm Durchmesser als Platzhalter eingebracht wird und somit die zentrale Hornhaut abflachen soll, was zu einer Korrektur von Kurzsichtigkeiten führt. Es werden zwei verschiedene Ringgeometrien beschrieben und entsprechende Dimensionsangaben gemacht. Ein kreisförmiger Ringquerschnitt mit einer Dicke von ca. 0,1mm bis 0,5mm und ein dreieckiger Ringquerschnitt mit Kantenlängen von ca. 0,3mm. Als Materialien werden im Wesentlichen polymere Kunststoffe genannt. Der Nachteil dieser Methode ist die hohe Traumatisierung des Gewebes. So muss nach Einbringen des Ringes in das Hornhautgewebe die Inzission entlang des gesamten Umfanges durch eine Naht verschlossen werden, da sonst der Ring nicht stabil in der Hornhaut gehalten werden könnte und die Stabilität der Hornhaut massiv beeinträchtigt würde. Ohne Naht wäre das Ausmaß der Korrektur, also die behandelten Dioptrien, daher nicht vorherbestimmbar. Aber auch mit einer Naht ist das Ausmaß der Korrektur nur dann einigermaßen vorherbestimmbar, wenn der Ring über eine ausreichende Steifigkeit und geringe Verformbarkeit verfügt um den möglichen Verziehungen des Gewebes durch die Naht ausreichenden Widerstand zu bieten.

Die WO 93/12735 A1 beschreibt eine Variante der GB 2095119 A mit einem biokompatiblen, ringförmigen Hornhautimplantat, welches ebenfalls durch eine zirkuläre Inzission von der Hornhautoberfläche in das Hornhautstroma zum Zwecke der Brechkraftkorrektur bei Kurzsichtigkeit eingebracht wird. Dabei handelt es sich um einen Ring mit fixem, also nicht veränderbaren Durchmesser, einem Brechungsindex, der nicht um mehr als 2% von dem des Hornhautgewebes abweicht und folgende Abmessungen aufweisen kann: Ringdurchmesser ca. 2,4mm bis 12 mm, Ringbreite ca. 0,2mm bis 4mm und Ringdicke ca. 0.005 bis 0.2 mm. Die Vorderseite des Ringes ist konvex und die Hinterseite eben ausgeführt. Auch hier ist die Traumatisierung des Gewebes bei der Anwendung erheblich. Des weiteren besteht ein erheblicher Nachteil für die Anwendung als Implantat in der Hornhaut durch den Verlauf der hinteren Fläche des Ringes. Diese ist gerade ausgeführt. Da jedoch die Hornhautoberfläche mit einem Radius von ca. 8 mm gekrümmt ist, führt dies dazu, dass bei den angegebenen Ringbreiten zum Teil erhebliche Verlaufsunterschiede zwischen der hinteren Ringfläche und der korrespondierenden Hornhautschnittfläche in einer Hornhauttasche auftreten. Ein solches geometrisches Missverhältnis kann, wie vielfach in der Literatur beschrieben, zu massiven Ablagerungen von organischem Material an den Grenzflächen führen, was zu Sehbeeinträchtigungen und zu schlechten kosmetischen Ergebnissen führen kann. Außerdem ergibt sich eine sehr ungleiche Druckverteilung auf das Gewebe entlang der Ringhinterfläche, was Druckatrophien und Gewebsuntergang induzieren kann.

Um die bislang beschriebenen Nachteile zu verhindern sind Verfahren entwickelt worden, bei welchen das jeweilige Hornhautimplantat, über einen schmalen, tunnelförmigen Zugang in eine sonst allseitig abgeschlossene Hornhauttasche eingebracht wird. Da die innere Spannung der Hornhaut entlang der Hornhautlamellen wirkt, wird bei einer im Wesentlichen geschlossenen Hornhauttasche die biomechanisch wirksame Querschnittsfläche für diese Spannung nicht reduziert, und die biomechanische Stabilität der Hornhaut nicht beeinträchtigt.

Eine geeignetes Verfahren und eine geeignete Vorrichtung zur Schaffung einer solchen Hornhauttasche ist beispielsweise aus der EP 1 620 049 B1 bekannt, deren Inhalt hiermit in diese Anmeldung aufgenommen wird.

Die Schaffung einer solchen Hornhauttasche samt, schmalen, mehr oder weniger tunnelförmigem Zugang ist aber auch bereits aus der US 2002/0055753 A1 bekannt. Nach dem Einbringen in die Hornhauttasche wird das gefaltete ringförmige Hornhautimplantat entfaltet und in Position gebracht. Obwohl die Implantation durch einen schmalen Tunnel mittels dieser sehr flexiblen, faltbaren Hornhautimplantate gut gelingt, ist ein wesentlicher Nachteil dieses bekannten Verfahrens und der dabei eingesetzten Hornhautimplantate jener, dass letztere nach der Implantation manuell in der Tasche entfaltet werden müssen. Dabei ist jedoch im Inneren der Tasche nach dem Einbringen des Hornhautimplantates eine Vielzahl von Kräften wirksam, die das Hornhautimplantat an der Entfaltung hindern, sodass besonders für sehr flexible Ringe das Risiko besteht, dass die Formgebung des Hornhautimplantates nach Implantation nicht mehr exakt die vordefinierte Ringform annimmt, und Astigmatismen bzw. auch Abbildungsfehler höherer Ordnung induziert werden. Es muss daher mit umständlichen und langwierigen manuellen Manipulationen nach der Implantation gerechnet werden, um die Ringform wieder herstellen zu können, was trotzdem oft nicht exakt möglich ist, wodurch die erwünschte Korrektur der Fehlsichtigkeit oft nicht erreicht wird. Außerdem bedingt das Erfordernis der manuellen Entfaltung des Hornhautimplantates innerhalb der Hornhauttasche eine unnötige Beanspruchung der Hornhaut und vor allem auch des schmalen, vorzugsweise tunnelförmigen Zugangs zur Hornhauttasche. Mit anderen Worten erleichtert ein aus dem Stand der Technik bekanntes, ausreichend flexibles Hornhautimplantat zwar die Einbringung in die Hornhauttasche durch den schmalen Zugang, erschwert aber gleichzeitig die Entfaltung in der Hornhauttasche.

Die WO 93/14703 A offenbart eine aus Hydrogel gefertigte Vorrichtung zum Dehnen der Iris des menschlichen Auges, welche in einem dehydrierten Zustand durch einen schlitzförmigen Zugang in die Iris eingesetzt wird und sich im implantierten Zustand mit Flüssigkeit sättigt, wodurch die Vorrichtung an Größe zunimmt und die Iris dehnt. Auf diese Weise soll einem Operateur ein besserer Zugang zu tiefer gelegenen Gewebebereichen des Auges ermöglicht werden.

Es ist daher die Aufgabe der vorliegenden Erfindung ein Hornhautimplantat zu schaffen, dass die beschriebenen Nachteile nicht aufweist und bei einem Verfahren zur Korrektur von Fehlsichtigkeit, bei welchem ein Hornhautimplantat in eine Hornhauttasche über einen schmalen, vorzugsweise tunnelförmigen Zugang eingebracht wird, einsetzbar ist.

### DARSTELLUNG DER ERFINDUNG

Erfindungsgemäß wird dies durch dies durch die kennzeichnenden Merkmale des Anspruchs 1 erreicht.

Dabei ist bei einem ringförmigen Hornhautimplantat zur Einbringung in eine Hornhauttasche des menschlichen Auges über einen schmalen, vorzugsweise tunnelförmigen Zugang, wobei die Endgestalt des Hornhautimplantats von der zu korrigierenden Fehlsichtigkeit abhängt, erfindungsgemäß vorgesehen, dass es ein aufgrund seiner Geometrie und/oder seines Materials aufgeprägtes Formengedächtnis besitzt, welches so ausgelegt ist, dass die Verformbarkeit aus einer Ausgangsgestalt die Einbringung des Hornhautimplantats in die Hornhauttasche über den tunnelförmigen Zugang ermöglicht und das Hornhautimplantat eine Einstellkraft in die Endgestalt aufweist, die das im wesentlichen selbständige Entfalten des Hornhautimplantats in der Hornhauttasche ermöglicht.

Gemäß einer bevorzugten Ausführungsform der Erfindung ist es weiters vorgesehen, dass Ausgangsgestalt und Endgestalt identisch sind.

Typischerweise sollte die Breite eines derartigen tunnelförmigen Zuganges zu einer sonst geschlossenen Hornhauttasche, um eine Beeinträchtigung der biomechanischen Stabilität der Hornhaut wie bei einem "flap" zu vermeiden, möglichst unter 5mm, idealerweise zwischen 2mm und 3mm betragen. Ein erfindungsgemäßes Hornhautimplantat kann daher auch durch einen schmalen Zugang, dessen größte lichte Breite unter 5mm, vorzugsweise zwischen 2mm und 3mm liegt, um eine Beeinträchtigung der biomechanischen Stabilität der Hornhaut wie bei einem "flap" zu vermeiden, in die Hornhauttasche eingebracht werden, ohne zu brechen oder formentfremdet (zum Beispiel durch bleibende (irreversible) plastische Deformation) zu werden.

Gleichzeitig kann aber auch gewährleistet werden, dass das Hornhautimplantat neben der ausreichenden Verformbarkeit, auch die Fähigkeit besitzt, eine ausreichende Einstellkraft aufzubringen, um sich nach der Implantation sicher in der Hornhauttasche in eine vorbestimmte Form - seine Endgestalt - entfalten zu können, wobei die Entfaltung im wesentlichen selbständig und automatisch, ohne zusätzlichen bzw. ohne nennenswerten zusätzlichen manuellen Eingriff erfolgt.

Ausschlaggebend dafür ist der Umstand, dass aufgrund des aufgeprägten Formengedächtnis dem erfindungsgemäßen Hornhautimplantat eine oder mehrere gewünschte Endgestalten "einprogrammiert" werden können und dieses, einmal einer Verformung unabhängig davon ob plastisch oder elastisch, ausgesetzt, entweder automatisch oder aber durch ein Triggersignal aktiviert, eine dieser Endgestalten annimmt.

Das Aufprägen des Formengedächtnis erfolgt dabei entweder aufgrund einer geeigneten Materialwahl oder aufgrund einer speziellen Geometrie des Hornhautimplantats oder aber aufgrund einer Kombination aus Material- und Geometrie.

Bei den einsetzbaren geeigneten Materialien kann es sich um elastisch oder nicht elastisch verformbare (plastische) Materialien handeln. Bei elastischen Materialien mit Formengedächtnis resultiert die Verformbarkeit und Einstellkraft vorwiegend aus der Elastizität des Materials, zB. PMMA (Polymethylmetakrylat), Silikon, etc. Bei nichtelastischen Materialien wie Formengedächtnislegierungen, resultiert die Einstellkraft z.B. aus atomaren Kräften, die bei der spontanen Umwandlung einer Gitterstruktur in eine andere frei werden.

Grundsätzlich gibt es eine Vielzahl an Materialien mit Formengedächtnis, die ein erfindungsgemäßes Formengedächtnis der Hornhautimplantate ermöglichen, so beispielsweise PMMA, Polymere aus EEMA oder HEMA oder andere Acrylmaterialien, Hydrogele, Nylon, Polycarbonat, polyethylen oder andere Kunststoffe, Kunststoffe mit temperaturabhängigem Formengedächtnis, Formengedächtnislegierungen (z.B. auf der Basis von NiTi, von Cu-Zn-Al, Cu-Al-Ni, etc.), geeignete Verbindungen von Kunststoffen mit Metallen oder Nichtmetallen (z.B. Keramiken, Halbleiter, etc.), geeignete Verbindungen von Metallen mit Nichtmetallen (z.B. Keramiken, Halbleiter, etc) oder Verbundwerkstoffe, , wobei der Einsatz einiger dieser Materialien, zB. Hydrogele, für Hornhautimplantate zwar bereits bekannt ist, jedoch erst die Einsatzbedingungen und insbesondere die Geometrie des Hornhautimplantats zur Aufprägung des gewünschten Formengedächtnisses führen.

Wichtig dabei ist, dass gewährleistet werden kann, dass die gewünschte Endgestalt aufgrund der Einstellkraft aus der Zwischengestalt, in die das Hornhautimplantat verformt werden muss, um durch den schmalen Zugang in die Hornhauttasche geschoben werden zu können, erreicht werden kann, unabhängig davon, ob Ausgangsgestalt und Endgestalt identisch sind oder nicht.

Die erfindungsgemäße Aufgabe kann aber auch, wie bereits erwähnt, ausschließlich oder zusätzlich durch Vorsehung einer speziellen Geometrie des Hornhautimplantates gelöst werden.

Dadurch können auch bekannte elastische Materialen als Hornhautimplantatkörper zum Einsatz kommen, die das gewünschte Formengedächtnis aufgrund ihrer geometrischen Dimensionierung erhalten.

Eine exakte Einstellung der Einstellkraft ist deswegen wichtig, da das Hornhautimplantat lediglich in seiner Endgestalt dazu geeignet ist, die jeweilige Fehlsichtigkeit zu korrigieren. Kann die Endstellung daher nicht erreicht werden bzw. nicht exakt erreicht werden, ist auch die Korrektur der Fehlsichtigkeit nur mangelhaft.

Auf das Hornhautimplantat wirken nach der Implantation in die Hornhauttasche erheblich Kräfte gegen die Entfaltung, wie zum Beispiel Reibungskräfte durch die Taschenwände, zwischen welchen das Hornhautimplantat zu liegen kommt. Verstärkt wird diese noch durch Kräfte die aus der intrinsischen Hornhautspannung und dem Augeninnendruck resultieren. Eine Aufprägung eines Formengedächtnisses alleine ist daher nicht ausreichend sondern muss zusätzlich gewährleistet sein, dass die Herstellung der - programmierten - Endgestalt mittels Einstellkraft erfolgt, welche geeignet ist, diese der Entfaltung in die Endgestalt entgegenwirkenden Kräfte zu überwinden.

Gemäß einer weiteren bevorzugten Ausführungsvariante der Erfindung handelt es sich bei einem geeigneten Material zur Aufprägung des Formengedächtnisses um Material mit aktivierbarem Formengedächtnis, vorzugsweise um ein Material mit elektrisch, thermisch, mechanisch oder magnetisch aktivierbarem Formengedächtnis.

Auf diese Art und Weise kann das Hornhautimplantat einmal implantiert jederzeit nachjustiert werden, wobei die Aktivierung auch zu unterschiedlichen Endgestalten führen kann.

So kann beispielsweise beim erfindungsgemäßen Einsatz von Materialien mit thermisch aktivierbarem Formengedächtnis das Hornhautimplantat auch nach plastischer Deformation seine gewünschte Endform annehmen. In einer bevorzugten Ausführungsvariante der Erfindung wird dies durch den gezielten Einsatz von Formengedächtnislegierungen erreicht.

Durch den erfindungsgemäßen Einsatz von Materialien mit aktivierbarem Formengedächtnis als Hornhautimplantat ist es des weiteren möglich, dass sich das in seiner Endgestalt befindliche, implantierte Hornhautimplantat auch nach Implantation z.B. durch Aktivierung mit einem von außen angelegten elektrischen oder magnetischen Feld oder durch Durchströmung mit elektrischem Strom in seiner Endgestalt noch weiter verändert.

Zur erfindungsgemäßen Gruppe der Materialien mit Formengedächtnis sind auch jene zu zählen, bei welchen durch Quellung oder Entquellung, z.B. durch Wasseraufnahme oder Wasserentzug die Form, die Abmessungen, oder die Elastizität, oder die Plastizität verändert werden kann. Solche Materialien sind z.B. vollständig oder unvollständig hydratisierte Kunststoffe wie HEMA oder Hydrogele.

Es sind aber auch Materialien mit Formengedächtnis bekannt, bei welchen die Aktivierung und Einstellung der Endgestalt durch mechanische (z.B. durch Applikation von Ultraschall oder Herabsetzung der Reibungskräfte in der Tasche) oder chemische Signale (z.B. Veränderung des pH-Wertes) erzielt wird.

Gemäß einer alternativen Ausführungsvariante der Erfindung handelt es sich bei den Materialien mit Formengedächtnis um Formengedächtnislegierungen, beispielsweise auf Basis NiTi,Cu-Zn-Al und Cu-Al-Ni, wobei gemäß einer bevorzugten Ausführungsvariante solche Hornhautimplantate mit einem inerten, biokompatiblen Schutzmantel ummantelt sind.

Um eine ganz besonders exakte automatische Entfaltung in der Hornhauttasche zu ermöglichen, ist es gemäß einer weiteren bevorzugten Ausführungsvariante der Erfindung vorgesehen, dass durch Auswahl eines geeigneten Materials sowie entsprechende Abstimmung mit der geometrischen Form des ringförmigen Hornhautimplantats erreicht wird, dass die Verformbarkeit des Hornhautimplantats mindestens 25% in zumindest einer Ringaußendimension vorzugsweise des Ringdurchmessers beträgt und das Hornhautimplantat eine Einstellkraft (bzw. Rückstellkraft) in die ursprüngliche Ringform (bzw. gewünschte Endgestalt) zwischen 0,001N und 1N, idealerweise zwischen 0,01N und 0,5N, in zumindest eine Richtung (die Richtung der Verformung), idealerweise aber in allen Richtungen aufweist.

Als besonders zu bevorzugende geometrische Form des ringförmigen Hornhautimplantats hat sich dabei ein kreisringförmiges Hornhautimplantat erwiesen, dessen Außendurchmesser zwischen 4mm und 12mm beträgt, dessen Ringbreite zwischen 0,4mm und 1,5mm vorzugsweise 0,5mm beträgt und dessen Ringhöhe zwischen 0,01mm und 0,8mm liegt. Dabei sollte der Innendurchmesser des Ringes möglichst nicht innerhalb der Pupillenweite liegen, um störende optische Randphänomene zu vermeiden.

Bevorzugterweise ist die vordere Oberfläche des Hornhautimplantats konvex und die rückwärtige Oberfläche konkav ausgebildet. Dadurch ist garantiert, dass die Taschenwand an praktisch jeder Stelle des Hornhautimplantats anliegt, wobei die vordere und die hintere Ringfläche an den Rändern möglichst ohne Stufe, auslaufend direkt ineinander übergehen.

Aufgrund der guten Verformbarkeit und erfindungsgemäß vorhandenen ausreichenden Einstellkraft kann das Hornhautimplantat auch als geschlossener oder gespaltener Ring ausgebildet sein und dennoch problemlos durch den schmalen Zugang in die Hornhauttasche geschoben werden. Die Ausbildung als geschlossener Ring garantiert, dass das Hornhautimplantat nach der Implantation in der Hornhauttasche wieder eine ringförmige, unverzerrte stabile Ringform einnehmen kann.

Das Hornhautimplantat kann Kreisringform als Endgestalt aufweisen, womit Kurzsichtigkeit korrigiert werden kann oder aber Nichtkreisringform womit andere Fehlsichtigkeiten wie beispielsweise Astigmatismus im Fall einer elliptischen Ringform als Endgestalt korrigiert werden können.

Zusätzliche bevorzugte Ausführungsvarianten sind den weiteren Unteransprüchen zu entnehmen.

### KURZE BESCHREIBUNG DER FIGUREN

Im Anschluss erfolgt nun eine detaillierte Beschreibung der Erfindung anhand von Ausführungsbeispielen. Dabei zeigt
- Fig.1: eine Schnittansicht durch ein erfindungsgemäßes Hornhautimplantat
- Fig.2: ein für die Einbringung in einen tunnelförmigen Zugang verformtes erfindungsgemäßes Hornhautimplantat
- Fig.3: ein erfindungsgemäßes Hornhautimplantat mit entlang des Umfangs unterschiedlichen Ringquerschnitten
- Fig.4: ein erfindungsgemäßes Hornhautimplantat mit sattelförmiger Ringgeometrie
- Fig.5: zwei erfindungsgemäße Hornhautimplantate mit unterschiedlicher Ringform
- Fig.6: unterschiedliche Querschnittsgeometrien erfindungsgemäßer Hornhautimplantate
- Fig.7: ein erfindungsgemäßes Hornhautimplantat in Form eines geschlossenen Kreisringes
- Fig.8: ein erfindungsgemäßes Hornhautimplantat in Form eines gespaltenen Kreisringes
- Fig.9: ein erfindungsgemäßes ringförmiges Hornhautimplantat mit zentralem Linsenkörper
- Fig.10: die Darstellung eines Implantationsvorganges eines erfindungsgemäßen ringförmigen Hornhautimplantates in eine Hornhauttasche über einen schmalen Tunnel

### WEGE ZUR AUSFÜHRUNG DER ERFINDUNG

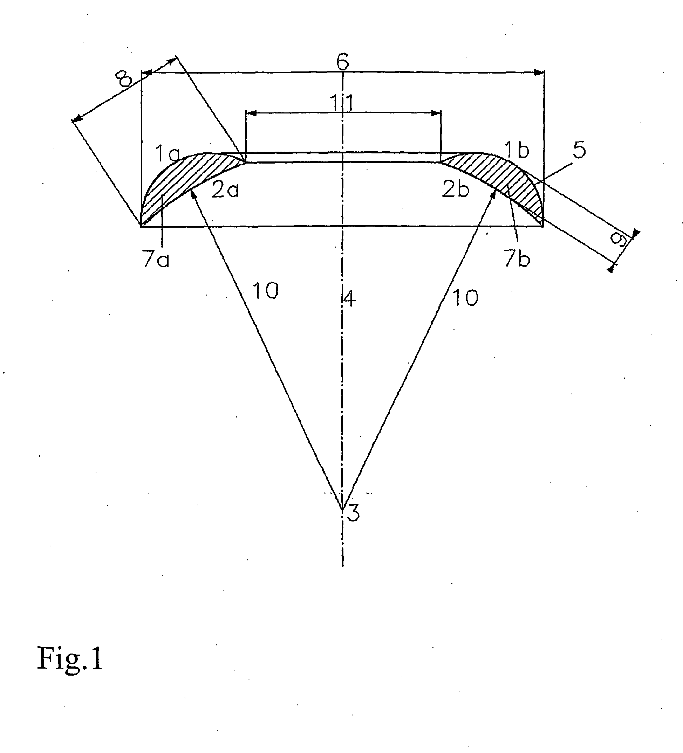

Wie in Fig.1 ersichtlich, besteht ein erfindungsgemäßes Hornhautimplantat aus einem ringförmigen Grundkörper 5, der eine vordere, konvexe Oberfläche 1 und eine rückwärtige, konkave Oberfläche 2 aufweist. Die Konkavität der rückwärtigen Oberfläche 2 kann sphärisch oder asphärisch, zB. durch Annäherung mittels mehrerer sphärischen Krümmungen, ausgeführt sein. Die Krümmungszentren 3 der Krümmungsradien 10 der beiden rückwärtigen Oberflächenabschnitte 2a,2b sind vorzugsweise entlang der Achse 4 des erfindungsgemäßen Hornhautimplantats angeordnet. Bei asphärischen Rückflächen sind mehrere Krümmungszentren 3 entlang der Achse 4, die der optischen Achse entspricht, angeordnet.

Dadurch wird nicht nur eine optimale Anpassung der Ringhinterfläche an das Implantationsbett erreicht und Einlagerungen an den Grenzflächen sowie Druckatrophien im Gewebe verhindert, auch die Verformbarkeit der Ringe wird so wesentlich erhöht, ohne die Einstellkraft in gleicher Weise zu reduzieren.

Der Außendurchmesser 6 des Hornhautimplantates liegt vorzugsweise zwischen 4mm und 12mm, idealerweise aber zwischen 5mm und 9mm, der Innendurchmesser 11 vorzugsweise zwischen 3mm und 11mm, idealerweise zwischen 4mm und 8mm. Die Ringbreite 8 beträgt idealerweise 0,5mm, sollte aus Nährstoffversorgungsgründen unter 1mm betragen und liegt bevorzugterweise zwischen 0,4mm und 1,5mm.

Die Ringhöhe 9 liegt in einem Bereich zwischen 0,01mm und 0,8mm, idealerweise zwischen 0.1mm und 0.4mm.

Der Innendurchmesser 11 des Hornhautimplantats sollte jedenfalls größer als die jeweilige Pupillenweite sein, um störende Randeffekte für den Patienten zu vermeiden.

Die vordere und die rückwärtige Oberfläche 1 und 2 gehen an den Rändern möglichst ohne Stufe, auslaufend ineinander über.

Idealerweise folgt die rückwärtige Oberfläche 2 dem natürlichen Verlauf der korrespondierenden Taschenwand in der Hornhaut (lokaler Hornhautradius minus Taschentiefe plus (oder mal) Korrekturfaktor, der die Deformation der Hornhaut durch die Einbringung in die Tasche berücksichtigt), sodass der konkave Verlauf der hinteren Ringfläche einer sphärischen oder asphärischen Krümmung, mit Radien 10 zwischen 4 mm und 40 mm entspricht, bevorzugterweise zwischen 6mm und 10mm.

Dem Hornhautimplantat 5 ist erfindungsgemäß aufgrund seines Materials und/oder seiner Geometrie ein Formengedächtnis aufgeprägt, welches so ausgelegt ist, dass das Hornhautimplantat einerseits eine Verformbarkeit aus einer Ausgangsgestalt aufweist, welche die Einbringung des Hornhautimplantats in die Hornhauttasche über den schmalen, vorzugsweise tunnelförmigen Zugang mit einer lichten Breite kleiner als 5mm ermöglicht und gleichzeitig eine Einstellkraft in die Endgestalt aufweist, die das im wesentlichen selbständige Entfalten des Hornhautimplantats in der Hornhauttasche ermöglicht.

Bevorzugte Materialien hierfür sind beispielsweise Kunststoffe wie PMMA, HEMA, Silikon, Polycarbonate, Polyethylen oder andere Polymere Kunststoffe oder Formengedächtnislegierungen.

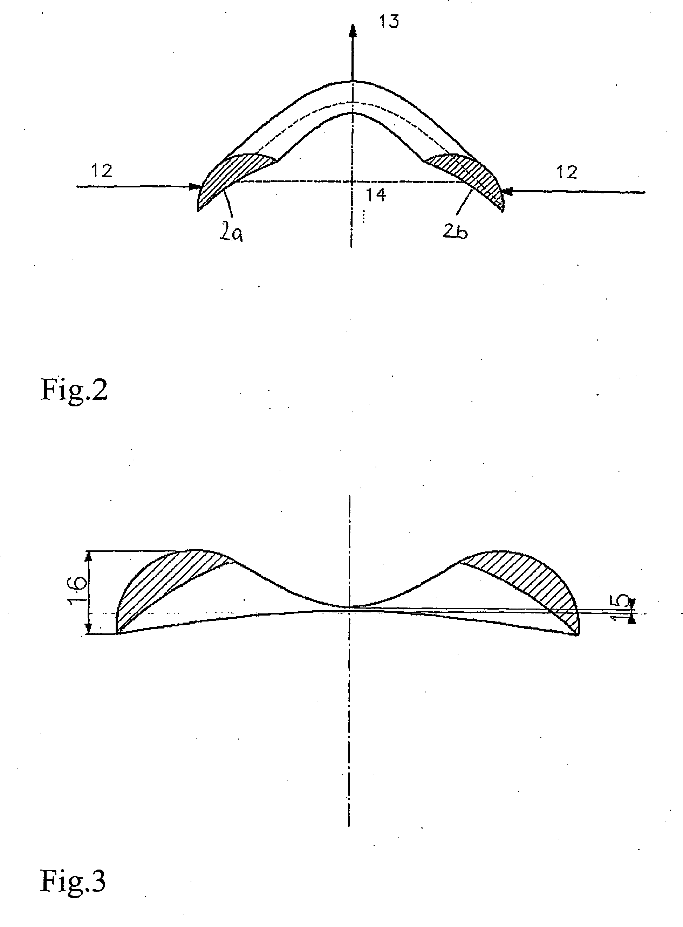

Fig.2 zeigt ein erfindungsgemäßes Hornhautimplantat, welches seitlich durch Krafteinwirkung symbolisiert durch die Pfeile 12, zusammengedrückt wird, um durch den (nicht gezeichneten) schmalen Zugang in eine Hornhauttasche eingeschoben werden zu können.

Im Allgemeinen hängt für den elastischen Verformungsbereich von Materialien die Einstellkraft umgekehrt proportional mit der Verformbarkeit zusammen.

Bei Verwendung von Materialien, die nur in einem sehr engen Verformungsbereich eine elastische Verformung ermöglichen (z.B. PMMA), ist es daher erforderlich, durch spezielle Formgebung des Hornhautimplantates eine ausreichende Verformbarkeit zu schaffen und trotzdem die Rückstellkraft aufrecht zu erhalten.

Im Falle eines Hornhautimplantates wie in Fig.2 dargestellt erfolgt dies durch den speziellen Verlauf der rückwärtigen Oberfläche 2 in Richtung Ringmittelpunkt indem die Krümmungszentren 3 der Radien 10 entlang der Achse 4 angeordnet sind, wodurch nicht nur eine simple ovale Gestalt erreicht wird, sondern ein sattelförmiges Gebilde, da ein Teil des Ringes aus der Ringebene bei der Verformung (Kompression) in die dritte Dimension 13 ausweicht. Dadurch wird ein weiterer Freiheitsgrad generiert, der die Verformbarkeit wesentlich erhöht, ohne die Einstellkraft wesentlich zu vermindern.

Erfindungsgemäß ist es vorgesehen, dass die Verformbarkeit mindestens 25% einer typischen Ringdimension beträgt und das Hornhautimplantat eine Einstellkraft in die ursprüngliche Ringform zwischen 0,001N und 1N, idealerweise zwischen 0,01N und 0,5N, aufweist.

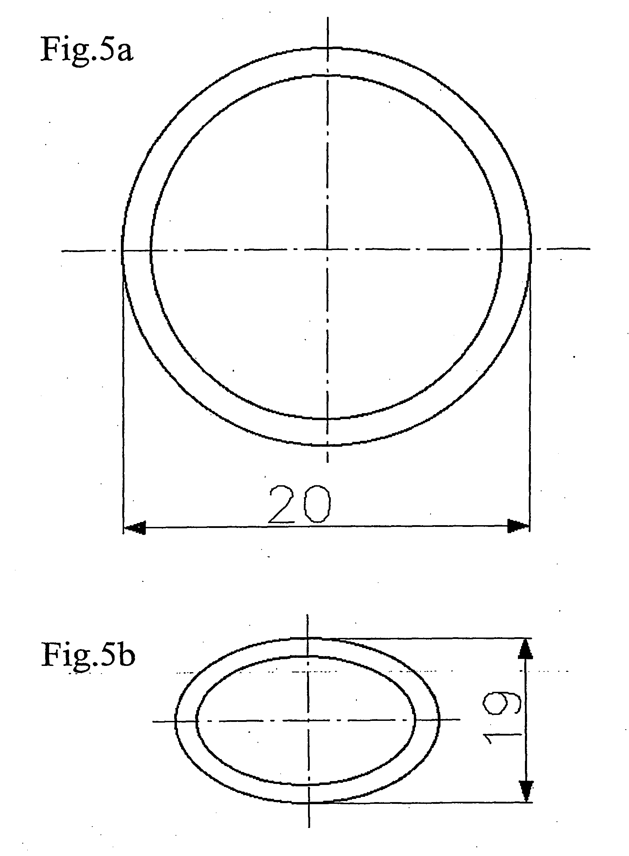

Eine typische Ringdimension bei einem kreisförmigen Hornhautimplantat ist beispielsweise der Ringdurchmesser 20, bei einem elliptischen Hornhautimplantat beispielsweise die kleine Hauptachse 19 (Fig.5).

Bei der Ausführung nach Fig.1 und Fig.2 ist es sogar möglich, einen Ring aus PMMA (ein Material das schwer verformbar ist und bei Verformung leicht bricht) mit 5mm Außendurchmesser 6, 0,5mm Ringbreite 8 und 0,25mm Ringhöhe 9 sowie einem Krümmungsradius 10 von 8mm auf ca. 50% seines Durchmessers zu verformen ohne dass er bricht und eine ausreichende Einstellkraft zur freien und selbstständigen Entfaltung in seine ursprüngliche Ringgeometrie nach Implantation in die Hornhauttasche gegen die Entfaltungswiderstände behält. Die Aufprägung des Formengedächtnisses erfolgt hier praktisch ausschließlich durch die Gestaltung einer speziellen Geometrie, wohingegen die Materialwahl bedeutungslos ist.

Um eine exakte Einstellung der Einstellkraft zu ermöglichen kann gegebenenfalls das Hornhautimplantat mit entlang des Umfangs unterschiedlichen Ringquerschnitten 15,16 ausgebildet sein, wie dies in Fig.3 gezeigt ist.

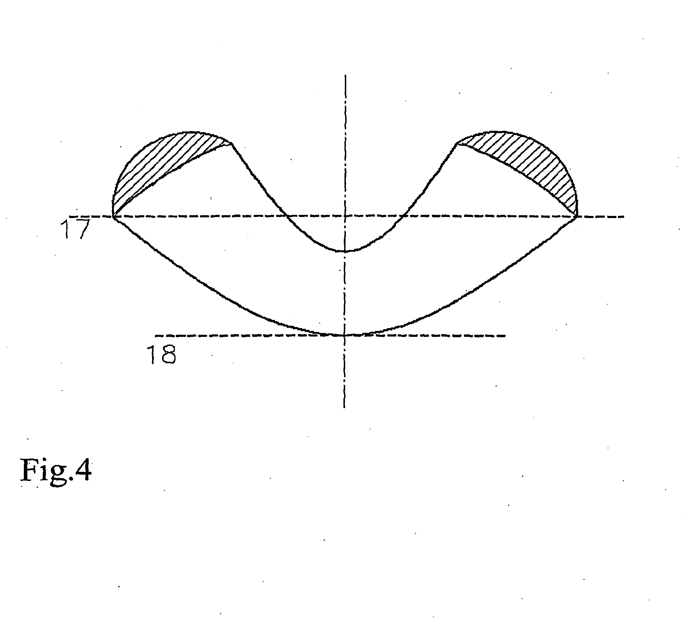

Im Falle der "Sattelform" befinden sich die Basiskanten 17, 18 entlang des Umfanges des Ringes nicht auf einer Ebene, wie dies aus Fig.4 ersichtlich ist.

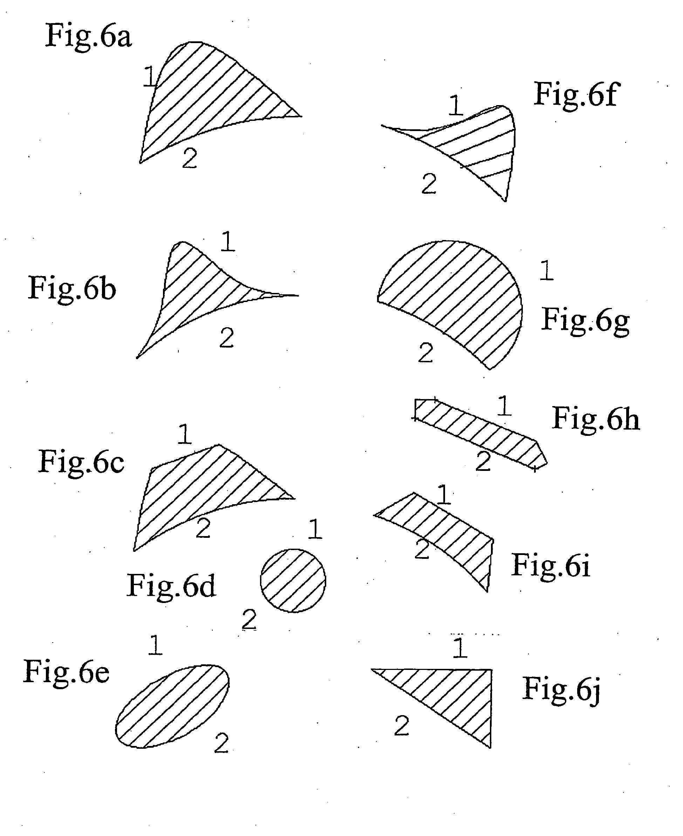

In Abhängigkeit der zu korrigierenden Fehlsichtigkeit kann das erfindungsgemäße Hornhautimplantat 5 aber auch unterschiedlichste Querschnittformen aufweisen, wie dies aus Fig.6 ersichtlich ist.

Durch die erfindungsgemäße exakte Abstimmung von Material und Geometrie des Hornhautimplantats 5 ist es gewährleistet, dass die Entfaltung des Hornhautimplantats in der Hornhauttasche nach der Implantation selbständig erfolgen kann.

Gegebenenfalls kann es sich bei dem Material um ein Material mit aktivierbarem Formengedächtnis handeln, vorzugsweise um ein Material mit elektrisch, thermisch, mechanisch oder magnetisch aktivierbarem Formengedächtnis, wie beispielsweise Formengedächtnislegierungen.

Als Triggersignal zur Aktivierung wird beispielsweise die Temperatur verwendet, so dass das erfindungsgemäße Hornhautimplantat in abgekühltem Zustand ausreichend verformbar ist, um durch den schmalen, vorzugsweise tunnelförmigen Zugang in die Hornhauttasche eingebracht werden zu können. In der Hornhauttasche erwärmt sich das Hornhautimplantat entsprechend bis zu einer gewissen Triggertemperatur, ab welcher das Hornhautimplantat wieder seine Endgestalt annimmt bzw. mit ausreichender Einstellkraft zwischen 0,001N und 1N, idealerweise zwischen 0,01N und 0,5N, die Endgestalt anstrebt. Da die Temperatur im Hornhautinneren unter Umständen nicht ausreicht um die Triggertemperatur zu erreichen, kann auch von außerhalb des Auges Wärme zugeführt werden.

Im Falle eines Materials, dass ein magnetisch oder elektrisch aktivierbares Formengedächtnis aufweist, kann das Material beispielsweise Elemente auf der Basis NiMnGa enthalten oder vollständig daraus bestehen. Dabei kann nach erfolgreicher Implantation in die Hornhauttasche im Sinne der Erfindung, eine Formanpassung von einer Endgestalt in eine andere Endgestalt durch Anlegen einer elektrischen Spannung an das Implantat oder durch Beaufschlagung mit einem elektrischen oder magnetischen Feld erfolgen. Dies ermöglicht sogar eine Feinjustierung oder Anpassung der Dioptrienkorrektur auch nachträglich, etwa wenn sich die Dioptrien des Auges im Laufe der Zeit wieder ändern sollten, ohne dass das Hornhautimplantat ausgetauscht werden muss.

Prinzipiell sind als Triggersignale zur Aktivierung des Formengedächtnisses verschiedene Varianten denkbar, so beispielsweise auch mechanische oder chemische Triggersignale. So kann bei Verwendung bestimmter Materialien, etwa auch bei geeignet elastisch verformbaren, durch Applikation von Ultraschall oder Druckausübung auf das Hornhautimplantat oder das Implantatbett die Annahme einer definierten Endgestalt befördert werden. Außerdem kann durch Veränderung des Quellungszustandes des Implantates, etwa durch Flüssigkeitszufuhr oder -entzug die Formgebung erleichtert werden bzw. eine entsprechende Aktivierungsenergie für die Formgebung überwunden werden. Desweiteren kann ein derartiges Triggersignal auch durch die gezielte Veränderung des pH-Wertes der Umgebung des Implantates im Gewebe oder des Hornhautimplantates selbst ausgelöst werden.

Typische Materialien mit aktivierbarem Formengedächtnis sind beispielsweise Polymermetalle, ionic polymer-metal composites, IMPC, elektroaktive Polymere (z.B. elektronische oder ionische EAP's)wie etwa polyacrylonitrile PAN, Keramiken oder elektroaktive Keramiken, geeignete Leiter- oder Halbleiterkunststoffe, ionic polymeric conductor composites, IPCC, magnetische Formengedächtnislemente z.B. auf der Basis von NiMnGa oder Ni2MnGa.

Formengedächtnislegierungen sind zum Beispiel Legierungen auf der Basis von NiTi, Cu-Zn-Al, Cu-Al-Ni, und anderen Materialien. Kunststoffe mit temperaturabhängigen Formengedächtnis sowie Formengedächtnislegierungen können vorzugsweise unterhalb einer Umwandlungstemperatur beliebig (insbesondere auch plastisch) verformt werden, nehmen aber oberhalb der Übergangstemperatur eine vorgegebene Form ein. Auf diese Weise kann durch Temperaturveränderung des zusammengefalteten Hornhautimplantates in Zwischengestalt nach Einführung in das stromale Implantationsbett die Entfaltung in die gewünschte Form erreicht werden.

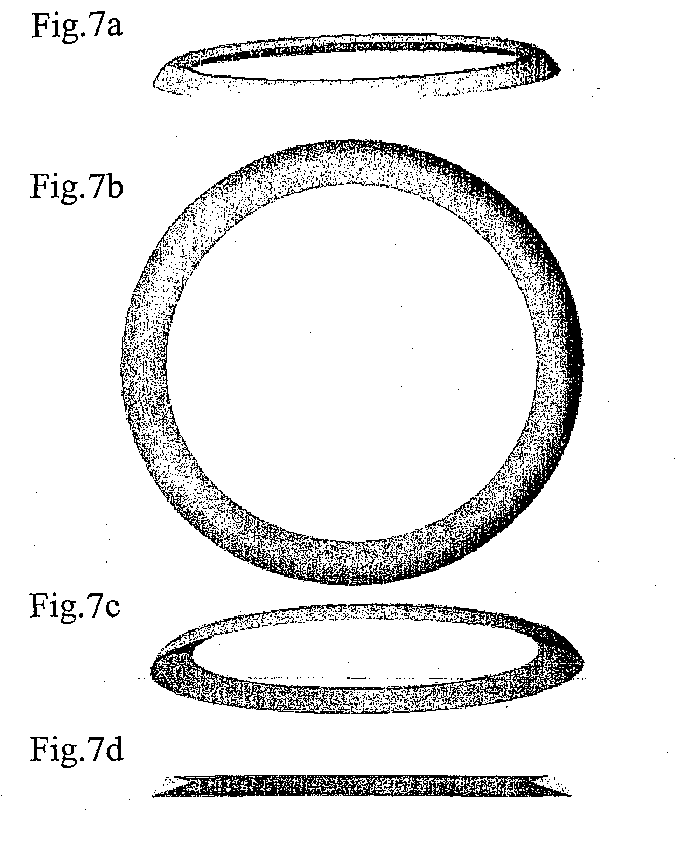

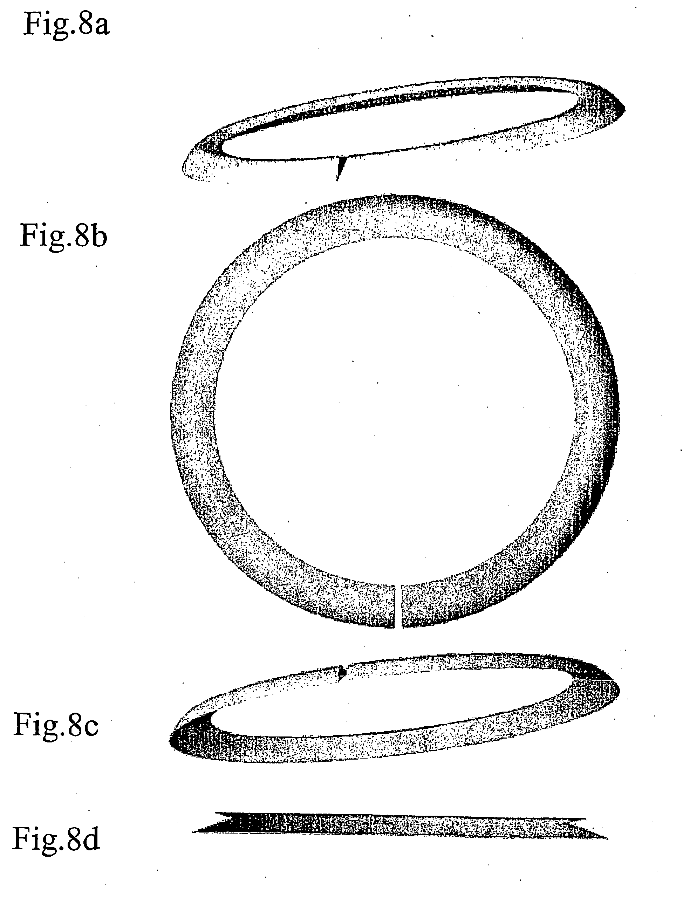

Formengedächtnislegierungen zeichnen sich durch einen martensitischen Phasenübergang (Phasenübergang 2. Ordnung) aus, wobei sich bei Überschreitung einer Umwandlungstemperatur die Kristallstruktur des Materials ändert und dabei eine vorher definierte Form annimmt. Es handelt sich dabei um ein atomares und kein molekulares Phänomen, wie etwa bei der elastischen Verformung, wo meist polymere Moleküle verformt werden. Vielmehr kommt es durch Überschreiten der Übergangstemperatur spontan zu einer völlig anderen Ordnung der Atome im Material. Obwohl manche Formengedächtnislegierungen biokompatibel sind, können Formengedächtnislegierungen etwa mit Silikon oder anderen inerten, biokompatiblen Materialien (z.B. Kunststoffen) ummantelt sein, damit das Gewebe nicht direkt der Legierung ausgesetzt ist und so Schaden nehmen kann. Solche Hornhautimplantate sind auch in der Lage einen Druck im Gewebe zu erzeugen, wenn Sie etwa an die Schnittgrenzen drücken, was zusätzlich zu einer beliebigen Verformung der Hornhautoberfläche beitragen kann. Die Form solcher Hornhautimplantate kann beliebig sein, insbesondere offen oder geschlossen ringförmig (siehe Fig.7 und 8), elliptisch, sattelförmig mit einem oder mehreren Satteln, spiralförmig, ein oder mehrgängig gewunden, gekrümmt oder gerade, o.ä. Die Implantate können mit oder ohne Kunststoffummantelung gefertigt sein. Sie können durchgehend oder segmentiert sein. Sie können mit elektrischen Kontaktelementen ausgestattet sein. Solche Elemente sind mehrheitlich auch für die Implantation in zirkuläre Tunnel geeignet.

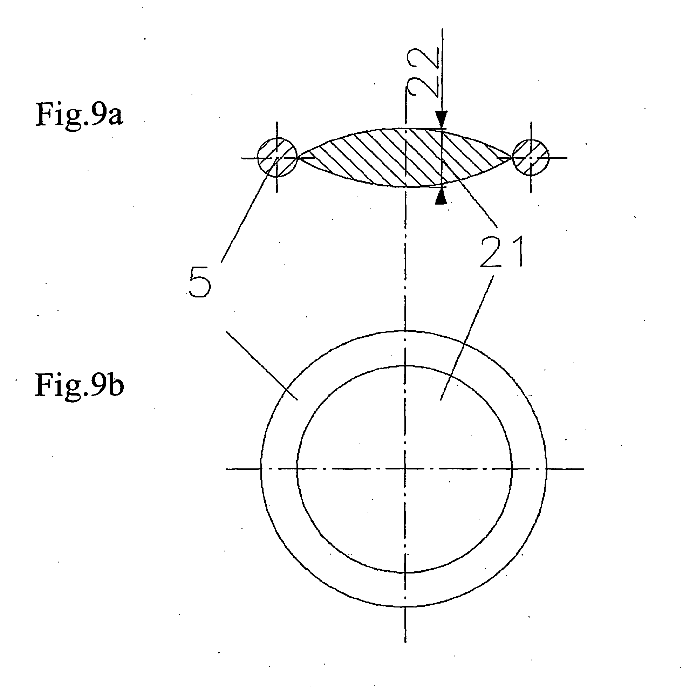

Fig.9 zeigt ein erfindungsgemäßes ringförmiges Hornhautimplantat mit einem zentralen Implantationskörper 21.

Bei der Korrektur der Weitsichtigkeit muss der zentrale Hornhautradius verkleinert werden. Daher muss ein zentraler Implantationskörper (Linse) 21 gewählt werden, der zentral einen größere Dicke aufweist als peripher, wobei die oben getätigten materialspezifischen Aussagen für ein ringförmiges Hornhautimplantat ohne zentralem Implantationskörper unverändert auch hier gelten. Insbesondere ist ein Hornhautimplantat hilfreich, welches zumindest teilweise aus einem Material mit Formengedächtnis bzw. Feinjustierbarkeit besteht.

Dabei ist eine zentrale möglichst verformbare Linse 21, vorzugsweise aus einem elastischen, transparenten, biokompatiblen Material von einem Ringkörper 5 mit Formengedächtnis umgeben. Durch eine Verbindung zwischen der zentralen Linse und dem umgebenden Ring kann die Linse gemeinsam mit dem Ring in die Hornhauttasche eingebracht und dort mit Hilfe des Ringes, in erfindungsgemäßer Weise entfaltet werden. Dies kann z.B. durch elastische Kräfte oder die Applikation einer bestimmten Temperatur oder einer elektrischen Spannung oder eines elektrischen oder magnetischen Feldes geschehen. Die Verbindung zwischen der zentralen Linse 21 und dem Ring 5 kann von beliebiger Art sein, z.B. durch Verschweißung, Einbringen in eine beide Elemente einschließende Folie, Verklebung, Einschmelzen des Ringes in die Linse, Integration des Ringes in die Linse auf jede beliebige Weise. Der zentrale Linsenkörper 21 kann beispielsweise aus Hydrogel, HEMA, Polyethylen, oder aus einem anderen polymeren oder nichtpolymeren Kunststoff bestehen. Wesentlich ist, dass er für Sauerstoff und/oder Nährstoffe ausreichend durchgängig ist. Des Weiteren kann er elastisch oder nichtelastisch ausgeführt sein. Der Brechungsindex kann beliebig sein. Der Linsenkörper 21 kann aber auch selbst ein Formengedächtnis aufweisen. Ausführungsvarianten wie für ein ringförmiges Hornhautimplantat ohne zentralem Linsenkörper 21 getätigt gelten ohne Einschränkungen auch für ringförmige Hornhautimplantate mit zentralem Linsenkörper 21.

Insbesondere kann der Ringkörper 5 aus allen hier beschriebenen Materialien gefertigt sein, so auch aus solchen, wo das Formengedächtnis thermisch oder elektrisch oder magnetisch aktivierbar ist. Damit kann auch z.B. durch einen elektrisch oder magnetisch nachjustierbaren Ringkörper (wie hier an anderer Stelle beschrieben) die Spannung und damit die zentrale Dicke (Mittendicke) 22 des elastischen Linsenkörpers 21 verändert und damit dessen Wirkung auf die Dioptrienkorrektur des Auges gesteuert werden. Durch geeignete Wahl der Geometrie des zentralen Linsenkörpers 21, z.B. einer diffraktiven oder refraktiven, bi-oder multifokalen Linse kann auch die Altersichtigkeit korrigiert werden. Er kann z.B. als Fresnelkörper ausgestattet sein. Es kann aber auch als teilweise oder vollständig intransparentes Medium ausgeführt sein. Er kann auch als Linsenkörper ohne direkte Dioptrienwirkung (z.B. ohne zentrale Verdickung 22) ausgeführt sein. Des Weiteren kann er bei intransparentem, z.B. schwarzem Material durch eine Abbildung mittels eines Einfach- oder Mehrfachspalt oder Einfach- oder Mehrfachloches (d.i. in bestimmter Weise angeordnete Löcher, so daß ein difraktiver Abbildungseffekt erzeugt wird) wirksam sein. Diese Löcher können durch die Anordnung diffraktiv wirksam sein oder z.B. bei nur einem solchen Loch stänopäisch wirksam sein. Diese Löcher müssen aber nicht physisch als solche ausgeführt werden, sondern können auch als transparente Bereiche in einem intransparenten Medium als solche wirken. Die getätigten Aussagen insbesondere für (offene) ringartige Strukturen oder Segmente gelten nicht nur auf Grundlage der hier beschriebenen Implantationstasche sondern auch für andere Implantationstaschen, insbesondere für vollständige und segmentierte ringförmige Taschen, wie sie etwa für die Implantation von Intacs verwendet werden.

Zur Korrektur von Astigmatismus sind asymmetrische Ringe (offen, geschlossen, geteilt oder segmentiert) oder Zentralkörper in Bezug auf Form oder Querschnitt notwendig. Ein myoper Astigmatismus wird daher am einfachsten entweder mit einem runden Implantat mit entlang den Hauptachsen unterschiedlichem Querschnittabmessungen oder mit homogenem Querschnitt aber vorzugsweise elliptischer Ringform oder einer Kombination von beiden korrigiert. Das gleiche gilt für den hyperopen Astigmatismus, nur dass dabei der Zentralkörper (Linse) anstatt des Ringes diese Asymetrien aufweist, welche ihm vorzugsweise durch einen der oben genannten Materialien und/oder Formen aufgeprägt wird. Im Besonderen sind dabei die zentralen Radien der beiden Hauptschnitte des astigmatischen Implantationskörpers unterschiedlich ausgeführt.

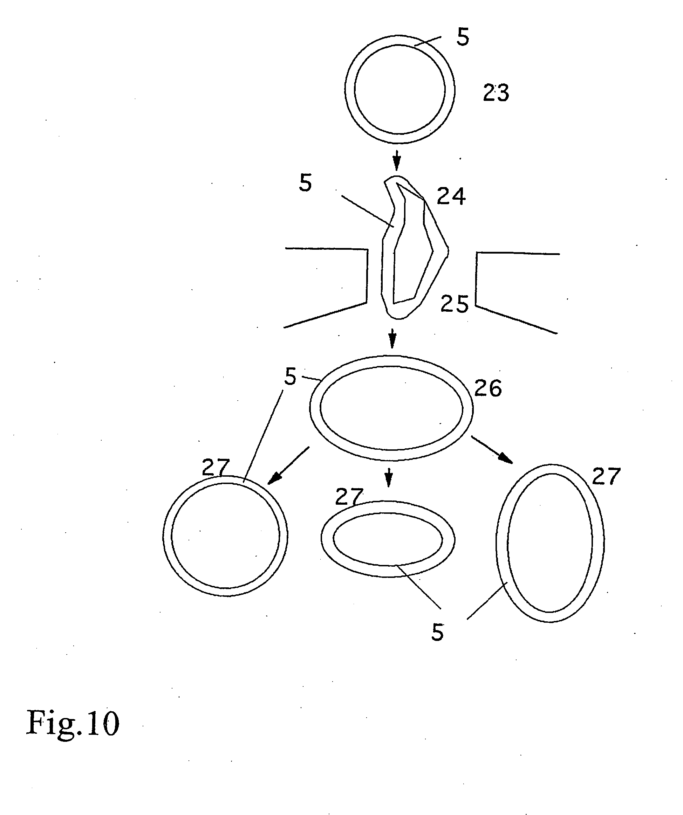

Fig.10 zeigt die Darstellung eines Implantationsvorganges eines erfindungsgemäßen ringförmigen Hornhautimplantates in eine Hornhauttasche über einen schmalen Tunnel. Dabei wird ein ringförmiges Hornhautimplantat 5 von einer Ausgangsgestalt 23, die beliebig sein kann, aber vorzugsweise der Endgestalt 26 oder 27 entspricht, in eine Zwischengestalt 24 zur Einbringung in eine Hornhauttasche über einen Tunnel 25 verformt. Anschließend wird durch das erfindungsgemäß aufgeprägte Formengedächtnis des Implantates 5 im Inneren der Hornhauttasche die Zwischengestalt 24 in eine vordefinierte. Gestalt 26 (Endgestalt) übergeführt, welche vorzugsweise mit der initialen Ausgangsgestalt 23 übereinstimmt aber dazu auch unterschiedlich sein kann. Dieser Übergang kann automatisch oder aber unmittelbar oder mittelbar über ein geeignetes Triggersignal erfolgen. Ein derartiges Triggersignal ist idealerweise eine Temperaturerhöhung des Implantates über eine bestimmte Umwandlungstemperatur. In weiterer Folge kann das Implantat in bestimmten Fällen von einer Endgestalt 26 in eine andere Endgestalt 27 übergeführt werden. Ausgelöst wird diese Umwandlung von einer Endgestalt 26 in eine andere Endgestalt 27 idealereweise durch elektrische oder magnetische Signale. Im besonderen kann die Endgestalt 27 von der applizierten elektrischen oder magnetischen Feldstärke oder vom elektrischen Strom durch das Implantat 5 abhängen. ,

Hervorzuheben ist, dass jedes Merkmal einer Ausführungsform mit jedem Merkmal einer anderen Ausführungsform kombiniert werden kann um eine neue Ausführungsform zu gestalten.

## Patentansprüche

1. Ringförmiges Hornhautimplantat (5) zur Einbringung in eine Hornhauttasche des menschlichen Auges über einen schmalen, vorzugsweise tunnelförmigen Zugang wobei die Endgestalt des Hornhautimplantats (5) von der zu korrigierenden Fehlsichtigkeit abhängt, **dadurch gekennzeichnet, dass** es ein aufgrund seiner Geometrie und/oder seines Materials aufgeprägtes Formengedächtnis besitzt, welches so ausgelegt ist, dass die Verformbarkeit aus einer Ausgangsgestalt die Einbringung des Hornhautimplantats (5) im die Hornhauttasche über den schmalen Zugang ermöglicht und das Hornhautimplantat (5) eine Einstellkraft in die Endgestalt aufweist, die das im wesentlichen selbständige Entfalten des Hornhautimplantats (5) in der Hornhauttasche ermöglicht.

2. Ringförmiges Hornhautimplantat (5) nach Anspruch 1, **dadurch gekennzeichnet, dass** Ausgangsgestalt und Endgestalt identisch sind.

3. Ringförmiges Hornhautimplantat (5) nach einem der Ansprüche 1 bis 2, **dadurch gekennzeichnet, dass** es sich bei dem Material um ein Material mit aktivierbarem Formengedächtnis handelt, vorzugsweise um ein Material mit elektrisch, mechanisch, thermisch oder magnetisch aktivierbarem Formengedächtnis.

4. Ringförmiges Hornhautimplantat (5) nach einem der Ansprüche 1 bis 3, **dadurch gekennzeichnet, dass** es sich bei dem Material um Formengedächtnislegierungen handelt, vorzugsweise auf Basis von NiTi, Cu-Zn-Al und Cu-Al-Ni.

5. Ringförmiges Hornhautimplantat (5) nach Anspruch 4, **dadurch gekennzeichnet, dass** das Hornhautimplantat (5) mit einem inerten, biokompatiblen Schutzmantel ummantelt ist.

6. Ringförmiges Hornhautimplantat (5) nach einem der Ansprüche 1 bis 5, **dadurch gekennzeichnet, dass** die Verformbarkeit mindestens 25% in zumindest einer Ringaußendimension vorzugsweise des Ringdurchmessers (6) beträgt und das Hornhautimplantat (5) eine Einstellkraft in die Endgestalt zwischen 0,001N und 1N, idealerweise 0,01N und 0,5N, aufweist.

7. Ringförmiges Hornhautimplantat (5) nach einem der Ansprüche 1 bis 6, **dadurch gekennzeichnet, dass** dessen Geometrie einen Außendurchmesser (6) zwischen 4mm und 12mm, eine Ringbreite (8) zwischen 0,4mm und 1,5mm vorzugsweise 0,5mm und eine Ringhöhe (9) zwischen 0,01mm und 0,8mm vorsieht.

8. Ringförmiges Hornhautimplantat (5) nach einem der Ansprüche 1 bis 7, **dadurch gekennzeichnet, dass** dessen vordere Oberfläche (1) konvex und die rückwärtige Oberfläche (2) konkav ausgebildet ist.

9. Ringförmiges Hornhautimplantat (5) nach einem der Ansprüche 1 bis 8, **dadurch gekennzeichnet, dass** es sich dabei um einen geschlossenen Ring handelt.

10. Ringförmiges Hornhautimplantat (5) nach einem der Ansprüche 1 bis 8, **dadurch gekennzeichnet, dass** es sich dabei um einen gespaltenen Ring handelt.

11. Ringförmiges Hornhautimplantat nach einem der Ansprüche 1 bis 10, **dadurch gekennzeichnet, dass** es entlang seines Umfangs in seiner Ringbreite (8) und Ringhöhe (9) variiert.

12. Ringförmiges Hornhautimplantat (5) nach einem der Ansprüche 1 bis 11, **dadurch gekennzeichnet, dass** es sich bei der Endgestalt um eine kreisrunde Ringform handelt.

13. Ringförmiges Hornhautimplantat (5) nach einem der Ansprüche 1 bis 12, **dadurch gekennzeichnet, dass** es sich bei der Endgestalt um eine nicht kreisrunde Ringform, vorzugsweise um eine elliptische Ringform handelt.

14. Ringförmiges Hornhautimplantat (5) nach einem der Ansprüche 1 bis 13, **dadurch gekennzeichnet, dass** der Innendurchmesser des Hornhautimplantates (5) kleiner als der photopische oder mesopische oder skotopische Pupillendurchmesser ist.

15. Ringförmiges Hornhautimplantat (5) nach einem der Ansprüche 1 bis 14, **dadurch gekennzeichnet, dass** innerhalb des Innendurchmessers (11) ein zumindest teilweise transparenter Zentralkörper (21) angeordnet ist.

## Claims

1. Annular cornea implant for inserting into a cornea pocket of the human eye via a narrow, preferably tunnel-shaped access, with the end shape of the cornea implant depending on the refractive error to be corrected, **characterised in that** it has a shape memory which is impressed on the basis of the geometry and/or material of the implant, and is designed in such a way that the deformability from a starting shape enables the insertion of the cornea implant into the cornea pocket via the narrow access, and the cornea implant has an adjustment force into the end shape thereof, which enables an essentially independent unfolding of the cornea implant in the cornea pocket.

2. Annular cornea implant according to claim 1, **characterised in that** the starting shape and the end shape are identical.

3. Annular cornea implant according to one of the claims 1 through 2, **characterized in that** the material is a material with activable shape memory, preferably a material with a shape memory that can be electrically, mechanically, thermally or magnetically activated.

4. Annular cornea implant according to one of the claims 1 through 3, **characterised in that** its material consists of shape memory alloys preferably based on Ni-Ti, Cu-Zn-Al und Cu-Al-Ni.

5. Annular cornea implant according to claim 4, **characterised in that** the cornea implant is enclosed by an inert, biocompatible protective layer.

6. Annular cornea implant according to one of the claims 1 through 5, **characterised in that** the deformability in at least one outer ring dimension, preferably the ring diameter, is at least 25 percent and the cornea implant has an adjustment force into the end shape in the range of 0.001N to 1N, ideally between 0.01N and 0.5N.

7. Annular cornea implant according to one of the claims 1 through 6, **characterised in that** its geometry comprises an outside diameter between 4mm and 12mm, a ring width between 0.4mm and 1.5mm, preferably 0.5mm, and a ring height between 0.01mm and 0.8mm.

8. Annular cornea implant according to one of the claims 1 through 7, **characterised in that** its front face is convex and its back face is concave.

9. Annular cornea implant according to one of the claims 1 through 8, **characterised in that** it is a closed ring.

10. Annular cornea implant according to one of the claims 1 through 8, **characterised in that** it is a split ring.

11. Annular cornea implant according to one of the claims 1 through 10, **characterised in that** its ring width and ring height vary along its circumference.

12. Annular cornea implant according to one of the claims 1 through 11, **characterised in that** the end shape is a circular ring shape.

13. Annular cornea implant according to one of the claims 1 through 12, **characterised in that** the end shape is not a circular ring shape, but preferably an elliptic ring shape.

14. Annular cornea implant according to one of the claims 1 through 13, **characterised in that** the inside diameter of the cornea implant is smaller than the photopic or mesopic or scotopic diameter of the pupil.

15. Annular cornea implant according to one of the claims 1 through 14, **characterised in that** within the inner diameter a central body 21 of at least partial transparency is arranged.

## Revendications

1. Implant dans la cornée en forme annulaire pour la mise dans une poche de cornée de l'oeil humain par un accès étroit, de préférence en forme d'un tunnel et que la forme finale de l'implant dans la cornée dépend de l'amétropie à corriger,**caractérisé par le fait qu'**il dispose à cause de sa géometrie et/ou de son matériel d'une mémoire de forme frappée qui est positionnée telle que la déformation d'une forme initiale permet la mise de l'implant de cornée dans la poche de cornée par un accés étroit et que l'implant de cornée présente une force de réglage qui permet en substance le dépliage indépendant de l'implant de cornée dans la poche de cornée.

2. Implant dans la cornée en forme annulaire selon la revendication 1 **caractérisé par le fait que** la forme initiale et la forme finale sont identiques.

3. Implant dans la cornée en forme annulaire selon l'une quelconque des revendications précédentes caractérise'par le fait que qu'il s'agit chez le matériel d'un matériel avec une mémoire de forme activable, de préférence d'un matériel avec une mémoire de forme électriquement, mécaniquement, thermiquement ou magnétiquement activable.

4. Implant dans la cornée en forme annulaire selon l'une des revendications précédentes **caractérisé par le fait que** qu'il s'agit chez le matériel des alliages de mémoire de forme, de préférence sur la base de NiTi, CU-Zn-Al et Cu-Al-Ni.

5. Implant dans la cornée en forme annulaire selon la revendication 4 **caractérisé par le fait que** l'implant est enveloppé d'un manteau de protection inert et biocompatible.

6. Implant dans la cornée en forme annulaire selon l'une quelconque des revendications 1 à 5, **caractérisé par le fait que** la déformation est au moins de 25 % dans au moins une dimension annulaire extérieure de préférence du diamètre de l'anneau et que l'implant dans la cornée présente une force de réglage dans la forme finale entre 0,001 N et 1 N, dans l'idéal 0,01 N et 0,5 N.

7. Implant dans la cornée en forme annulaire selon l'une quelconque des revendications 1 à 6 , **caractérisé par le fait que** sa géometrie prévoit un diamètre extérieur entre 4mm et 12mm, une largeur d'anneau entre 0,4mm et 1,5mm, de préférence 0,5mm et une hauteur d'anneau entre 0,01mm et 0,8mm.

8. Implant dans la cornée en forme annulaire selon l'une quelconque des revendications 1 à 7, **caractérisé par le fait que** sa surface antérieure est convexe et sa surface de l'arrière est concave.

9. Implant dans la cornée en forme annulaire selon l'une quelconque des revendications 1 à 8, **caractérisé par le fait qu'**il s'agit d'un anneau fermé.

10. Implant dans la cornée en forme annulaire selon l'une quelconque des revendications 1 à 8, **caractérisé par le fait qu'**il s'agit d'un anneau fendu.

11. Implant dans la cornée en forme annulaire selon l'une quelconque des revendications 1 à 10, **caractérisé par le fait qu'**il varie dans sa largeur et de sa hauteur le long de sa circonférence.

12. Implant dans la cornée en forme annulaire selon l'une quelconque des revendications 1 à 11, **caractérisé par le fait qu'**il s'agit chez la forme finale d'une forme annulaire circulaire.

13. Implant dans la cornée en forme annulaire selon l'une quelconque des revendications 1 à 12 , **caractérisé par le fait qu'**il s'agit chez la forme finale pas d'une forme annulaire ciculaire, de préférence d'une forme annulaire elliptique.

14. Implant dans la cornée en forme annulaire selon l'une quelconque des revendications 1 à 13 , **caractérisé par le fait que** le diamètre intérieur de l'implant dans la cornée est plus petit que le diamètre photopique ou mésopique ou skotopique de la pupille.

15. Implant dans la cornée en forme annulaire selon l'une quelconque des revendications 1 à 14, **caractérisé par le fait qu'**un corps central au moins partiellement transparent 21 est arrangé dans le diamètre intérieur.
